# EUROPÄISCHE PATENTSCHRIFT

(11) **EP 0 804 501 B1**
(45) Veröffentlichungstag und Bekanntmachung des Hinweises auf die Patenterteilung: **22.05.2002**
(21) Anmeldenummer: 96900923.2
(22) Anmeldetag: 09.01.1996
(51) Int. Cl.: C08K 5/15

(54) **MIKROKAPSELN, ENTHALTEND EIN STABILISATORGEMISCH AUS CHROMANDERIVATEN, INERTEN ORGANISCHEN LÖSUNGSMITTELN UND ORGANISCHEN PHOSPHITEN ODER PHOSPHONITEN UND/ODER AMINEN.**
MICROCAPSULES CONTAINING A STABILISER MIXTURE MADE FROM CHROMANE DERIVATIVES, INERT ORGANIC SOLVENTS AND ORGANIC PHOSPHITES OR PHOSPHONITES AND/OR AMINES.
MICROCAPSULES CONTENANT UN MELANGE STABILISATEUR CONTENANT DES DERIVES DE CHROMANE, DES SOLVANTS ORGANIQUES INERTES, DES PHOSPHITES OU PHOSPHONITES ORGANIQUES ET/OU AMINES.

(30) Priorität: 16.01.1995 DE 19501053
(43) Veröffentlichungstag der Anmeldung: 05.11.1997
(73) Patentinhaber: BASF AKTIENGESELLSCHAFT, 67056 Ludwigshafen (DE)
(72) Erfinder: TRAUTH, Hubert, D-67373 Dudenhofen (DE); KROCKENBERGER, Jürgen, D-70569 Stuttgart (DE); JAHNS, Ekkehard, D-69469 Weinheim (DE); BIASTOCH, Ralf, D-67346 Speyer (DE)
(86) Internationale Anmeldenummer: EP9600058
(87) Internationale Veröffentlichungsnummer: WO9622325

(56) Entgegenhaltungen:
- EP-A- 0 198 089
- EP-A- 0 312 927
- EP-A- 0 457 154
- EP-A- 0 542 108
- WO-A-93/10178
- US-A- 4 404 304
- US-A- 4 680 327
- US-A- 4 806 580
- US-A- 5 232 769
- STN International, File CAPLUS, CAPLUS accession no. 1980:424764, Kriger, G. et al: "Effect of vitamins E and D2 on the stability of carotene as a function of the solvent and the carotene and vitamin concentration"; & LLA Raksti (1979) 168, 51-60
- STN International, File CAPLUS, CAPLUS accession no. 1994:550706, Iwatsuki, Misato et al: "Effects of solvents and media on the antioxidant activity of .alpha.-tocopherol"; & Biochim. Biophys. Acta (1994), 1200(1), 19-26
- PATENT ABSTRACTS OF JAPAN Bd. 009, Nr. 108 (C-280) 11 Mai 1985 & JP 60 001 125 A (EISAI KK) 07 Januar 1985
- DATABASE WPI Week 198507, Derwent Publications Ltd., London, GB; Class B05, AN 1985-041564 & JP 60 001 125 A (EISAI CO LTD) 07 Januar 1985
- PATENT ABSTRACTS OF JAPAN Bd. 018, Nr. 262 (C-1201) 19 Mai 1994 & JP 06 040 891 A (BIZEN KASEI KK) 15 Februar 1994
- DATABASE WPI Week 199411, Derwent Publications Ltd., London, GB; Class B07, AN 1994-089265 & JP 06 040 891 A (BIZEN KASEI KK) 15 Februar 1994

## Beschreibung

Die vorliegende Erfindung betrifft Mikrokapseln, enthaltend ein Stabilisatorgemisch aus Chromanderivaten, inerten organischen Lösungsmitteln und organischen Phosphiten oder Phosphoniten und/- oder Aminen zur Stabilisierung von organischem Material gegen die Einwirkung von Licht, Sauerstoff und insbesondere von Wärme.

Aus der DE-A 36 34 531 (1) sind Stabilisatorgemische zur Stabilisierung von Kunststoffen aus Chromanderivaten (Vitamin E, α-Tocopherol) und organischen Phosphiten bzw. Phosphoniten bekannt. Die Mischungen haben jedoch den Nachteil, daß sie sowohl bei der Lagerung als auch nach der Einarbeitung in die Kunststoffe nicht stabil sind. Wahrscheinlich aufgrund von Hydrolysereaktionen in Gegenwart von Spuren von Luftfeuchtigkeit beobachtet man eine Abnahme des Gehaltes an Chromanderivaten und damit eine Verringerung der stabilisierenden Wirkung auf die Kunststoffe.

In der deutschen Patentanmeldung P 44 05 670 (2) wird ein Stabilisatorgemisch aus Chromanderivaten, organischen Phosphiten bzw. Phosphoniten und Aminen zur Stabilisierung von organischem Material, insbesondere von Kunststoffen, empfohlen, jedoch weist auch dieses Gemisch noch nicht die optimale Stabilität bei der Einarbeitung in die Kunststoffe auf.

Die genannten Stabilisatorgemische des Standes der Technik haben weiterhin den großen Nachteil, daß sie flüssig oder breiartig sind und sich somit äußerst schlecht mit thermoplastischen Kunststoffen mischen lassen. Die meisten Kunststoffhersteller und -verarbeiter können darüber hinaus derartige Additive nicht flüssig dosieren und fordern feste Additive, die gut wägbar und leicht handhabbar und zumischbar sind.

Aufgabe der vorliegenden Erfindung war es daher, ein stabiles Stabilisatorgemisch bereitzustellen, welches außerdem als Feststoff einsetzbar ist.

Demgemäß wurden Mikrokapseln, enthaltend ein Stabilisatorgemisch, enthaltend
(a) ein oder mehrere Chromanderivate der allgemeinen Formel I 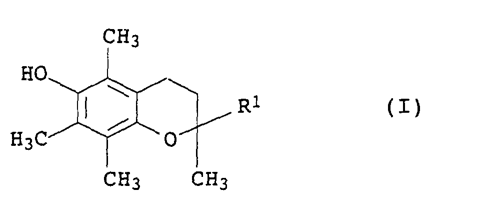 in der R¹ eine Gruppe der Formel 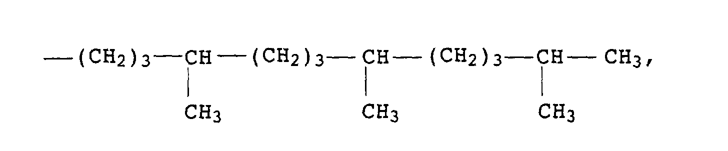 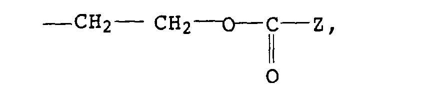 worin Z für C₇- bis C₃₀-Alkyl steht,
   -CH₂CH₂-S-(C₁- bis C₃₀-Alkyl) oder 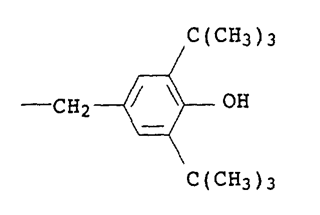 bedeutet,
(b) ein inertes organisches Lösungsmittel für die Chromanderivate I aus der Gruppe der aliphatischen, cycloaliphatischen oder aromatischen Kohlenwasserstoffe oder Halogenkohlenwasserstoffe, der Siliconöle und der pflanzlichen und tierischen fetten öle,
(c) ein oder mehrere organische Phosphite der allgemeinen Formel II 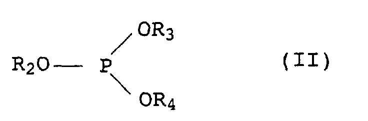 in der die Reste R² bis R⁴ jeweils C₂- bis C₁₂-Alkyl oder C₆- bis C₁₈-Aryl, welches durch C₁- bis C₁₈-Alkylgruppen substituiert sein kann, bezeichnen,
   oder ein organisches Phosphonit der Formel III 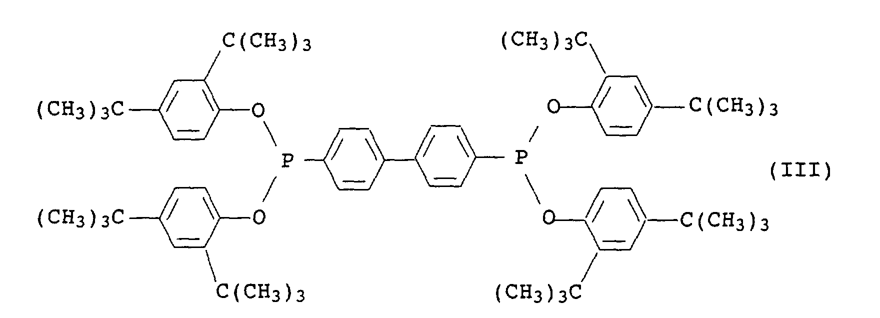 oder Mischungen aus den Phosphiten II und dem Phosphonit III und/- oder
(d) ein oder mehrere Amine der allgemeinen Formel IV 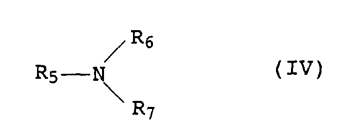 in der die Reste R⁵ bis R⁷ jeweils für Wasserstoff, für C₁- bis C₁₈-Alkyl, welches durch bis zu 5 nicht benachbarte Sauerstoffatome oder Gruppen der Formel -NR⁸- unterbrochen sein und durch bis zu 3 Hydroxylgruppen substituiert sein kann, wobei R⁸ Wasserstoff oder C₁- bis C₄-Alkyl bedeutet, oder für Phenyl, welches durch bis zu 3 C₄- bis C₁₈-Alkylgruppen substituiert sein kann, steht, mit der Ausnahme von NH₃ als Amin IV,
wobei die Komponenten (a) und (b) im Gewichtsverhältnis von 99:1 bis 1:99 und die Komponenten (b) und (c) im Gewichtsverhältnis von 100:0 bis 1:99 stehen und die Komponente (d) in einer Menge von 0 bis 2,0 Gew.-%, bezogen auf die Menge von (a)+(c), im Stabilisatorgemisch vorliegt, mit der Maßgabe, dass mindestens eine der Komponenten (c) oder (d) anwesend ist, gefunden.

Als Chromanderivate I eignen sich vor allem 2,5,7,8-Tetramethyl-2-(2'-stearoyloxyethyl)chroman (R¹= -CH₂CH₂-O-CO-C₁₇H₃₅) und insbesondere α-Tocopherole, vorzugsweise DL-α-Tocopherol (R¹= -(CH₂)₃-CH(CH₃)-(CH₂)₃-CH(CH₃)-(CH₂)₃-C(CH₃)₂).

Als inerte organische Lösungsmittel (b) für die Chromanderivate I eignen sich vor allem
- Benzin, Mineralöl und Paraffine wie Weißöl
- partiell hydrierte Terphenyle
- Toluol, Xylole und C₂- bis C₁₈-Alkylbenzole wie Dodecylbenzol
- C₁- bis C₁₂-Alkylnaphthaline
- Chlorparaffine
- Fluorkohlenwasserstoffe
- übliche Polysiloxane wie Polydimethylsiloxane oder Poly(methylphenylsiloxane)
- natürlich vorkommende Triglyceride gesättigter und/oder ungesättigter Fettsäuren wie Sojaöl, Rüböl, Olivenöl, Sonnenblumenöl, Baumwollsaatöl, Erdnußöl, Leinöl, Rapsöl oder Fischöl

Diese inerten organischen Lösungsmittel (b) sind normalerweise bei den üblichen Einarbeitungstemperaturen von Additiven in Kunststoffe, d.h. bis ca. 300°C, wärmebeständig. Sie weisen in der Regel eine geringe Flüchtigkeit auf und haben insbesondere Siedetemperaturen über 80°C.

Die organischen Lösungsmittel (b) können auch in Form von Mischungen verschiedener der genannten Species eingesetzt werden.

Die einsetzbaren organischen Phosphite II sind sowohl flüssige wie auch kristalline Produkte. Als Beispiel für derartige Phosphite sind zu nennen:
- Trisalkylphosphite mit bevorzugt langkettigen linearen oder verzweigten Alkylgruppen wie Octyl-, Nonyl-, Isononyl-, Decyloder Isodecylgruppen;
- Trisarylphosphite mit unsubstituierten oder ein- bis dreifach alkylsubstituierten Arylgruppen wie Phenyl-, Nonylphenyl- oder 2,4-Di-tert.-butylphenylgruppen;
- gemischte Arylalkylphosphite, wie Diisodecylphenylphosphit oder Diphenylpentaerythritdiphosphit.

Die Phosphite der Formel II können nach bekannten Methoden synthetisiert werden, beispielsweise durch Umsetzung von PCl₃ mit ein- oder mehrwertigen Alkoholen in Gegenwart einer organischen Base oder mit gegebenenfalls substituierten Phenolen oder Lösungsmittel bei 20 bis 250°C. Die gemischten Alkylarylphosphite werden beispielsweise durch Umsetzung von Triphenylphosphit mit ein- oder mehrwertigen Alkoholen in Gegenwart eines basischen Katalysators, vorzugsweise ohne Lösungsmittel, hergestellt.

Das Phosphonit III ist bekannt und im Handel unter dem Namen Irgafos® P-EPQ der Firma Ciba-Geigy erhältlich.

Die einsetzbaren Amine IV können primäre, sekundäre vorzugsweise tertiäre Amine sein.

Als Beispiele für derartige Amine seien genannt: Butylamin, Dibutylamin, Tributylamin, Tripropylamin, Triisopropylamin, Octylamin, Diisobutylamin oder Stearylamin.

Bevorzugt werden weiterhin Amine, die Hydroxylgruppen enthaltende C₂- bis C₁₈-Reste für R⁵ bis R⁷ besitzen, z.B. Ethanolamin, Diethanolamin, Triethanolamin, Propanolamin, Dipropanolamin, Tripropanolamin, Isopropanolamin, Diisopropanolamin und insbesondere Triisopropanolamin.

Die Amine IV sollten allerdings eine nicht zu hohe Flüchtigkeit aufweisen, daher eignet sich Ammoniak (NH₃) nicht für das Stabilisatorgemisch.

Es können also Stabilisatorgemische aus den Komponenten (a)+(b) +(c), (a)+(b)+(d) oder (a)+(b)+(c)+(d) als den wesentlichen Bestandteilen vorliegen.

Die Komponenten (a) und (b) stehen vorzugsweise im Gewichtsverhältnis von 90:10 bis 5:95, insbesondere 60:40 bis 10:90, stehen.

Bei Mitanwesenheit von (c) beträgt das Gewichtsverhältnis (b) zu (c) vorzugsweise 95:5 bis 5:95; es hat sich als günstig erwiesen, wenn gleichzeitig das Gewichtsverhältnis (a) zu (c) im Bereich von 1:1 bis 1:14, insbesondere 1:5 bis 1:10, liegt. Bei Mitanwesenheit von (d) liegt diese Komponente vorzugsweise in einer Menge von 0,001 bis 2,0 Gew.-%, insbesondere 0,01 bis 1,0 Gew.-%, vor allem 0,02 bis 0,5 Gew.-%, jeweils bezogen auf die Menge von (a)+(c), im erfindungsgemäßen Stabilisatorgemisch vor.

Als Wandmaterial (Hüllen) dieser Mikrokapseln dienen üblicherweise feste polymere Materialien natürlichen oder vorzugsweise synthetischen Ursprungs, im Kern enthalten sie das flüssige oder breiartige Stabilisatorgemisch, welches in der Regel 50 bis 95 Gew.-%, insbesondere 70 bis 90 Gew.-% des Gesamtgewichtes der Mikrokapsel ausmacht.

Im Stand der Technik sind Mikrokapseln bekannt, deren Hüllen aus Polykondensaten auf Basis von Harnstoff, Phenol oder insbesondere Melamin und Formaldehyd bestehen. So wird beispielsweise in der EP-B 026 914 (3) ein Verfahren zur Herstellung solcher Mikrokapseln durch Kondensation von Melamin-Formaldehyd-Vorkondensaten oder deren C₁- bis C₄-Alkylethern in Wasser, in dem das wasserunlösliche, den Kapselkern bildende Material dispergiert ist, in Gegenwart von gelösten Polymeren mit negativ geladenen ionischen Gruppen, insbesondere sulfonsäuregruppenhaltigen Polymeren, beschrieben. Die so erhältlichen Mikrokapseln werden zur Herstellung von druckempfindlichen Aufzeichnungspapieren eingesetzt. Das in (3) beschriebene Herstellungsverfahren für Mikrokapseln eignet sich ebenfalls hervorragend zur Herstellung der erfindungsgemäßen Mikrokapseln, welche das Stabilisatorgemisch aus den Komponenten (a), (b) und (c) und/oder (d) enthalten.

Als Ausgangsstoffe für das Wandmaterial eignen sich vorzugsweise Melamin-Formaldehyd-Vorkondensate und deren C₁- bis C₄-Alkylether mit einem Gewichtsverhältnis von Melamin zu Formaldehyd von vorzugsweise 1:3 bis 1:6. Diese Vorkondensate sind N-Methylolmelaminverbindungen oder deren Ether mit Alkanolen. Die für das Verfahren verwendeten Vorkondensate sollen mit Wasser in jedem Verhältnis mischbar sein, ohne eine Trübung zu erzeugen. Durch Abkühlung auftretende Trübungen müssen sich durch Erwärmen entfernen lassen. Aus diesen Gründen sind die Ether der Methylolmelamine besonders bevorzugt.

Als waserlösliche Sulfonsäuregruppen tragende Polymere kommen z.B. Homo- oder Copolymerisate des Sulfoethyl(meth)acrylats, des Sulfopropyl(meth)acrylats, der Maleinimid-N-ethansulfonsäure oder 2-Acrylamido-2-methylpropansulfonsäure in Betracht. Bevorzugt sind Polymere der 2-Acrylamido-2-methylpropansulfonsäure, die sich leicht zu Polymeren mit den gewünschten K-Werten polymerisieren läßt. Die Polymeren liegen in Form der freien Säure oder vorzugsweise der Alkalimetall- oder trisubstituierten Ammoniumsalze vor. Als Sulfonsäuregruppen tragende Polymere kommen außerdem Copolymere in Betracht, die aus den genannten Sulfongruppen tragenden Monomeren oder Vinylsulfonsäure und C₁- bis C₃-Alkylacrylaten, Hydroxy-C₂- bis C₄-alkylacrylaten wie Methyl-, Ethyl-, n- oder iso-Propylacrylat, Hydroxypropylacrylat und/oder N-Vinylpyrrolidon aufgebaut sind. Im Falle der Acrylate beträgt deren Anteil im Copolymerisat maximal 30 Gew.-%. Im Falle der Hydroxyalkylacrylate soll deren Anteil nicht größer als 10 Gew.-%, bezogen auf die Summe der Comonomeren, sein. Bei Copolymerisaten mit N-Vinylpyrrolidon liegt der Anteil an Sulfonsäuregruppen tragenden Monomeren bei mindestens 5, vorzugsweise bei 30 Gew.-% und darüber, bezogen auf die Summe der Comonomeren. Von den Copolymerisaten sind solche mit 2-Acrylamido-2-methylpropansulfonsäure als sulfonsäuregruppentragende Comonomere bevorzugt. Die sulfonsäuregruppentragenden Homo- und Copolymerisate werden nach bekannten Verfahren hergestellt.

Die Polymeren sollen einen K-Wert nach Fikentscher von 100 bis 170 (gemessen in 1 gew.-%iger wäßriger Lösung bei 20°C) oder eine Viskosität von 200 bis 5000 mPas bei einem Schergefälle von 489 s⁻¹ (gemessen bei 25°C in 20 gew.-%iger wäßriger Lösung bei pH 4,0 bis 7,0) aufweisen. Bevorzugt sind Polymere mit einem K-Wert von 115 bis 160, deren Viskosität 400 bis 4000 mPas beträgt.

Die Anwendungsmenge an wasserlöslichen Sulfonsäuren enthaltenden Polymeren liegt in der Regel zwischen 1 und 5,5, vorzugsweise zwischen 1,5 und 4,5 Gew.-%, bezogen auf die wäßrige Phase.

Die optimale Menge der wasserlöslichen Sulfogruppen enthaltenden Polymeren wird einmal vom Polymeren selbst, zum anderen von der Reaktionstemperatur, der gewünschten Mikrokapselgröße und dem Vorkondensat aus Melamin und Formaldehyd beeinflußt. Durch einfache Versuche kann die optimal benötigte Menge leicht ermittelt werden. Es hat sich herausgestellt, daß die optimale Konzentration des wasserlöslichen Sulfogruppen aufweisenden Polymeren praktisch unabhängig ist vom Verhältnis der wäßrigen kontinuierlichen Phase zur organischen, wasserunlöslichen Kernmaterialphase. Das heißt, unter den einmal optimierten Bedingungen können Mikrokapseldispersionen mit variablen Gehalten an Kapseln bei praktisch gleichbleibender Qualität hergestellt werden.

Die Weiter- und Auskondensation der Vorkondensate während und nach der Kapselbildung erfolgt zweckmäßigerweise bei pH-Werten von 3,0 bis 6,5, vorzugsweise von 3,5 bis 5,5. Der pH-Wert in der wäßrigen Phase kann mit Säuren, wie Schwefelsäure, Salzsäure, Salpetersäure, Phosphorsäure, Oxalsäure oder vorzugsweise Ameisensäure, oder bei saurer wäßriger Phase mit Natronlauge eingestellt werden. Der Beginn der Trübung, d.h. die Ausfällung des Melamin-Formaldehyd-Kondensates, ist etwas vom Vorkondensat abhängig, so daß es für die Bildung der Mikrokapseln aus verschiedenen Vorkondensaten etwas unterschiedliche optimale pH-Werte und Temperaturen gibt.

Im allgemeinen sind für das beschriebene Verfahren Temperaturen von 15 bis 100°C zweckmäßig, bevorzugt sind Temperaturen von 40 bis 90°C, um eine schnellere Mikrokapselbildung zu erreichen.

Die Weiterkondensation des Vorkondensates kann im Bereich der oben genannten pH-Werte und Temperaturen in Abwesenheit, aber auch in Anwesenheit des wasserlöslichen Sulfogruppen tragenden Hochpolymeren erfolgen. Letzteres Verfahren ist bevorzugt, da die sich aus der wäßrigen Lösung ausscheidenden Weiterkondensatteilchen, die die Trübung verursachen, dann von gleichmäßigerer Größe sind.

Das hydrophobe zu verkapselnde Material, d.h. das Stabilisatorgemisch, kann entweder nach dem Auftreten der Trübung zugegeben werden oder bereits während der Weiterkondensation des Vorkondensates anwesend sein.

Gemäß dem beschriebenen Verfahren sind Mikrokapseln verschiedener Durchmesser herstellbar. So werden die Kapseln im allgemeinen kleiner, wenn man mehr Vorkondensat oder hydrophiles Schutzkolloid (d.h. die sulfogruppenhaltigen Polymeren) einsetzt oder intensiver dispergiert oder die Verweilzeit in der Dispergierstufe verlängert. Größere Kapseln sind entsprechend mit den umgekehrten Maßnahmen, einzeln oder in Kombination, zu erhalten. Der Weiterkondensationsgrad des Vorkondensates ist von Einfluß auf die Kapselgröße. Liegt er im Optimum, werden unter sonst konstanten Bedingungen die kleinsten Kapseln erhalten. Im allgemeinen werden Kapseln mit Durchmessern von 1 bis 200 µm, insbesondere solche von 2 bis 50 µm, erhalten.

Die Mikroverkapselung wird in der Regel diskontinuierlich gemäß (3) durchgeführt. Die Mikroverkapselung kann aber auch kontinuierlich erfolgen, z.B. wie in der EP-B 218 887 (4) beschrieben.

Der bei der Kondensation der Melamin-Formaldehyd-Vorkondensate freiwerdende Formaldehyd kann gebunden werden, z.B. in bekannter Weise mit Ammoniak bei pH-Werten oberhalb 7 bis 8 oder mit Harnstoff oder Ethylenharnstoff. Besonders vorteilhaft ist, bei den erhaltenen Dispersionen den freien Formaldehyd durch Kondensation mit Melamin zu binden. Hierzu wird die noch von der Kondensation her saure Dispersion der Teilchen bei 60 bis 90°C, bevorzugt bei 70 bis 85°C, und bei pH 4,0 bis 5,0 unter Rühren innerhalb einer halben bis 2 Stunden mit einer Aufschlämmung von Melamin in Wasser (Gewichtsverhältnis Melamin zu Wasser vorzugsweise 1:2 bis 1:4) kontinuierlich versetzt, wobei man die Kondensation bis zum Verbrauch des freien Formaldehyds fortführt.

Die nach dem Verfahren erhaltenen Dispersionen können getrocknet werden, z.B. in einem Sprühtrockner. Die dabei erhaltenen Pulver sind frei von Agglomeraten und lassen sich in Wasser oder Lösungsmittel enthaltende Systeme einfach einarbeiten. Für die Isolierung durch Sprühtrocknung sind vor allem die vollständig von Vorkondensat befreiten Dispersionen geeignet.

Neben dem oben beschriebenen Melaminharz-Verfahren können auch andere Verfahren zur Herstellung der erfindungsgemäßen Mikrokapseln eingesetzt werden, so beispielsweise Verfahren gemäß der EP-A 457 154 (5) unter Verwendung von C₁- bis C₂₄-Alkylestern der Acryl- oder Methacrylsäure, gemäß der EP-A 468 323 (6) durch Grenzflächenpolyaddition oder Grenzflächenpolykondensation zwischen einem Hydroxylamin und einer mit Amino- und Alkoholgruppen reaktiven Komponente, z.B. Isocyanat, oder gemäß der US-A 5 064 470 (7) unter Verwendung von Gelatine, beispielsweise in Kombination mit Gummi arabicum.

Besonders gut geeignet sind pulverförmige Mikrokapseln, um mit Hilfe eines Extruders als Feststoff gut dosierbar in einen Kunststoff eingearbeitet zu werden. Auf diese Weise können die Stabilisatorgemische in Form der getrockneten Mikrokapseldispersionen mit einem Kunststoffgranulat homogen vorgemischt werden und ohne Verklebungen oder andere Maschinenstörungen im Extruder verarbeitet werden.

Die erfindungsgemäßen Mikrokapseln eignen sich in hervorragender Weise zum Stabilisieren von organischem Material, insbesondere von Kunststoffen, gegen die Einwirkung von Licht, Sauerstoff und vor allem Wärme. Sie sind auch wirksam als Metalldesaktivatoren. Sie werden dem zu stabilisierenden organischen Material in einer Konzentration von 0,005 bis 10,0 Gew.-%, vorzugsweise von 0,01 bis 4,0 Gew.-%, insbesondere von 0,05 bis 2,0 Gew.-%, bezogen auf das organische Material, vor oder während seiner Herstellung zugesetzt.

Als Kunststoffe, die durch die erfindungsgemäßen Mikrokapseln stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copoymerisate von Mono- oder Diolefinen oder Mischungen der genannten Polymeren;
Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS);
Halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyphenylenether, Polyester, Polycarbonate, Polysulfone, Polyethersulfone und Polyetherketone.

Als gutstabilisierbare Kunststoffe kommen insbesondere Thermoplaste wie Polyvinylchlorid, Styrolpolymerisate, Polyamide, Polycarbonate, Polyphenylenoxid, Polyester, Polyolefine, vorzugsweise Polyethylen und Polypropylen, Polyurethane sowie Duroplaste in Betracht.

Für die Eignung und Wirksamkeit der erfindungsgemäßen Mikrokapseln ist neben der geringen Eigenfarbe und der Verarbeitungsstabilität vor allem die Hydrolysebeständigkeit und der stabile Gehalt an Chromanderivaten I besonders wichtig.

Unter organischem Material sind weiter beispielsweise photographische Aufzeichnungsmaterialien, insbesondere photographische Emulsionen, Vorprodukte für Kunststoffe und Lacke oder Lacke selbst zu verstehen.

Zur Vermischung der erfindungsgemäßen Mikrokapseln vor allem mit Kunststoffen können alle bekannten Vorrichtungen und Methoden zum Einmischen von Stabilisierungsmitteln oder anderen Zusätzen in Polymere angewandt werden.

Die erfindungsgemäßen Mikrokapseln können vor allem zur Stabilisierung von Kunststoffen bei deren Verarbeitung verwendet werden. Derartige Mikrokapseln werden Kunststoffen während oder vor der Verarbeitung zugesetzt, um die Kunststoffe vor Zersetzung zu schützen, wobei sich bekanntlich die Wirkungen verschiedener Stabilisatorsysteme addieren können.

Neben dem mikroverkapselten Stabilisatorsystem aus den Komponenten (a), (b) und (c) und/oder (d) können so auch weitere Stabilisatorzusätze, z.B. die für Stabilisierungszwecke bekannten Synergisten Calciumstearat und Distearylthiodipropionat (S-(CH₂CH₂-COOC₁₈H₃₇)₂), in üblichen Mengen in die Kunststoffe mit eingemischt werden.

Mit den beschriebenen Stabilisatoren können auch zusammen mit Kunststoffen Konzentrate hergestellt werden und diese dann zusammen mit den zu stabilisierenden Kunststoffen verarbeitet werden. Bei der Verarbeitung bringen je nach Anwendungsgebiet Konzentrate Vorteile, da diese bei der Verarbeitung leichter zu handhaben und zu dosieren sind.

Gegenstand der vorliegenden Erfindung ist außerdem gegen die Einwirkung von Licht, Sauerstoff und vor allem Wärme stabilisiertes organisches Material, insbesondere Kunststoffe, welches die erfindungsgemäßen Mikrokapseln in den oben angegebenen Konzentrationen enthält.

Das beschriebene Verfahren zur Herstellung der erfindungsgemäßen Mikrokapseln soll durch das folgende Ausführungsbeispiel weiter erläutert werden. Im folgenden beziehen sich die Teile und Prozente auf das Gewicht. Dabei sind die Prozentangaben ihrerseits auf das Gewicht der Lösung oder Dispersion bezogen. Volumenteile entsprechen Gewichtsteilen mit der Dichte 1.

Der angegebene Feststoffgehalt wurde durch Trocknung (4 Stunden bei 105°C) bestimmt und setzt sich im wesentlichen aus den Mikrokapseln und dem wasserlöslichen Polymeren zusammen. Der Siebrückstand wurde durch Sieben der Dispersion über ein Rüttelsieb mit 40 µm-Maschenweite erhalten und feucht gewogen. Er enthält dann ca. 50 % Wasser. Die Kapseldurchmesser wurden subjektiv unter dem Mikroskop, objektiv mittels eines Malvern Auto-Sizers bestimmt. Angegeben wird der Kapseldurchmesser in den für die häufigste Teilchengröße (Zahlenmittel) und für die Teilchenfraktion mit dem größten Gesamtvolumen (Volumenmittel) sowie die Halbwertsbreite des Volumenmittels als der Kapseldurchmesser bzw. die Kapseldurchmesserdifferenz (HW), die in der differentiellen Verteilungskurve bei 50 % der Häufigkeit ausgewiesen wird.

Die Viskosität der Kapseldispersion wird als Auslaufzeit in Sekunden von 100 ml Dispersion aus dem DIN-Becher mit 4 mm-Düse angegeben. Die Viskosität der 20 %igen Lösungen der wasserlöslichen stark saure Gruppen, z.B. Sulfonsäure, enthaltenden Polymeren wurde bei 25°C im Rheomat® 30 (Firma Contraves) bei einem Schergefälle von 489 sec⁻¹ gemessen. Der K-Wert wurde nach Fikentscher (Cellulosechemie 13 (1932), 58 ff), 1 %ig in Wasser, bei 20°C bestimmt.

### Beispiel 1

In einem zylindrisch geformten 4-1-Rührgefäß mit einem Dissolverrührer mit 5 cm-Zahnscheibe wurden 1000 g Wasser, 141 g einer 70 %igen, wäßrigen Lösung eines in Wasser klar löslichen, partiell methylierten Vorkondensates (enthielt ca. 2,3 CH₃O-Gruppen pro Melaminmolekül) aus 1 mol Melamin und 5,25 mol Formaldehyd, 129 g einer 20 %igen wäßrigen Lösung von Poly-2-acrylamido-2-methylpropansulfonsäure-Natriumsalz (K-Wert = 140) vorgelegt. Bei 3000 Upm Rührgeschwindigkeit wurde bei 30°C eine Lösung aus 93 g DL-α-Tocopherol und 93 g Tris-nonylphenylphosphit in 525 g Weißöl hinzugegeben und dispergiert. Der pH-Wert wurde jetzt mit Ameisensäure auf 3,6 eingestellt. Nach einer Stunde Dispergieren wurde der Ansatz mit einem Propellerrührer bei 800 Upm für eine Stunde bei 30°C weitergerührt. Anschließend wurde unter Rühren auf 80°C aufgeheizt und für 2 Stunden bei dieser Temperatur gehalten. Anschließend wurde die Mikrokapseldispersion mit einer 50 %igen Lösung von Triethanolamin in Wasser auf einen pH-Wert von 7,5 neutralisiert.

Die Mikrokapseln hatten eine Teilchengröße von 7 bis 20 µm nach lichtmikroskopischer Betrachtung. Der Feststoffgehalt der Dispersion betrug 39,5 %. 2000 g der Dispersion wurden in einem Sprühtrockner mit einer Eingangstemperatur von 140°C und einer Ausgangstemperatur von 65-70°C getrocknet. Die Ausbeute war 800 g eines freifließenden Pulvers mit einem Feststoffgehalt von 96,7 %.

### Anwendungsbeispiel

Für die Eignung und Wirksamkeit von Kunststoffstabilisatoren ist vor allem die Verarbeitungsstabilität von Bedeutung.

Das mikroverkapselte Stabilisatorgemisch aus Beispiel 1 wurde in einem Extruder in additivfreies dechloriertes Polypropylen homogenisiert und granuliert. Aus dieser einmal extrudierten Probe wurde der Schmelzindex nach DIN 53 735 bestimmt. Diese Probe wurde dann noch viermal extrudiert und granuliert und nach der dritten und fünften Extrusion der Schmelzindex des Extrudats bestimmt.

Die Meßergebnisse sind in der folgenden Tabelle zusammgestellt.

Dabei ist der extrudierte Kunststoff von um so besserer Qualität, je niedriger der Schmelzindexwert ist.

**Tabelle**

| Stabilisatorgemisch gemäß | Konzentration des Stabilisatorgemisches im Polypropylen | Schmelzindex nach der | | |
|---|---|---|---|---|
| . Beispiel Nr | [Gew.-%] | 1. | 3. | 5. |
| | | Extrusion | | |
| 1 | 0,10 | 25 | 32 | 45 |
| Zum Vergleich: ohne Stabilisator | - | 39 | 77 | >150 |

## Patentansprüche

1. Mikrokapseln, enthaltend ein Stabilisatorgemisch, enthaltend
(a) ein oder mehrere Chromanderivate der allgemeinen Formel I 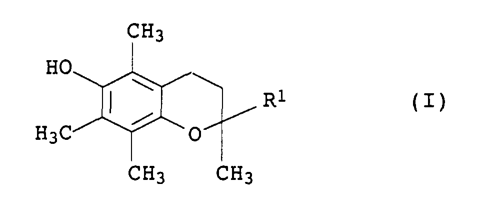 in der R¹ eine Gruppe der Formel 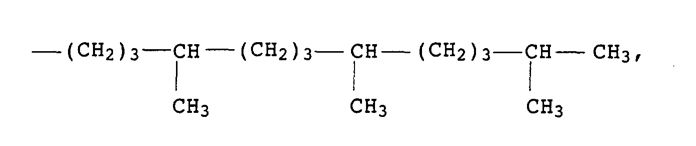 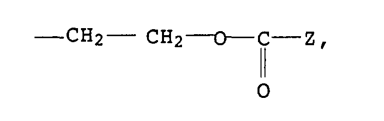 worin Z für C₇- bis C₃₀-Alkyl steht,
-CH₂CH₂-S-(C₁- bis C₃₀-Alkyl) oder 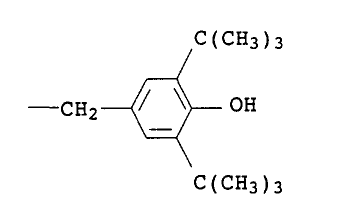 bedeutet,
(b) ein inertes organisches Lösungsmittel für die Chromanderivate I aus der Gruppe der aliphatischen, cycloaliphatischen oder aromatischen Kohlenwasserstoffe oder Halogenkohlenwasserstoffe, der Siliconöle und der pflanzlichen und tierischen fetten Öle,
(c) ein oder mehrere organische Phosphite der allgemeinen Formel II 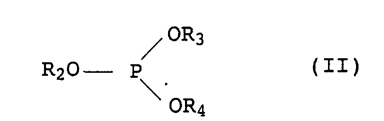 in der die Reste R² bis R⁴ jeweils C₂- bis C₁₂-Alkyl oder C₆- bis C₁₈-Aryl, welches durch C₁- bis C₁₈-Alkylgruppen substituiert sein kann, bezeichnen,
oder ein organisches Phosphonit der Formel III 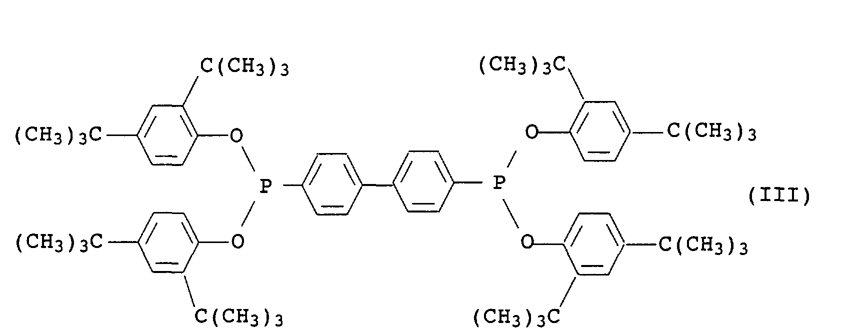 oder Mischungen aus den Phosphiten II und dem Phosphonit III und/oder
(d) ein oder mehrere Amine der allgemeinen Formel IV 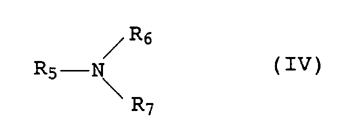 in der die Reste R⁵ bis R⁷ jeweils für Wasserstoff, für C₁- bis C₁₈-Alkyl, welches durch bis zu 5 nicht benachbarte Sauerstoffatome oder Gruppen der Formel -NR⁸- unterbrochen sein und durch bis zu 3 Hydroxylgruppen substituiert sein kann, wobei R⁸ Wasserstoff oder C₁- bis C₄-Alkyl bedeutet, oder für Phenyl, welches durch bis zu 3 C₄- bis C₁₈-Alkylgruppen substituiert sein kann, steht, mit der Ausnahme von NH₃ als Amin IV,
wobei die Komponenten (a) und (b) im Gewichtsverhältnis von 99:1 bis 1:99 und die Komponenten (b) und (c) im Gewichtsverhältnis von 100:0 bis 1:99 stehen und die Komponente (d) in einer Menge von 0 bis 2,0 Gew.-%, bezogen auf die Menge von (a)+(c), im Stabilisatorgemisch vorliegt, mit der Maßgabe, dass mindestens eine der Komponenten (c) oder (d) anwesend ist.

2. Verwendung von Mikrokapseln gemäß Anspruch 1 zum Stabilisieren von organischem Material gegen die Einwirkung von Licht, Sauerstoff und Wärme.

3. Gegen die Einwirkung von Licht, Sauerstoff und Wärme stabilisiertes organisches Material, enthaltend 0,005 bis 10,0 Gew.-%, bezogen auf die Menge des organischen Materials, Mikrokapseln gemäß Anspruch 1.

## Claims

1. Microcapsules containing a stabilizer mixture comprising
(a) one or more chroman derivatives of the general formula I 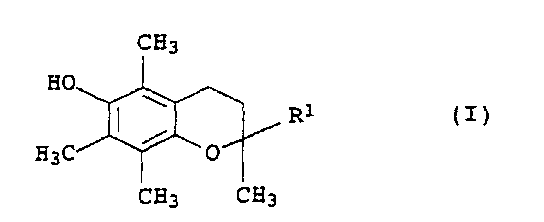 in which R¹ is a group of the formula 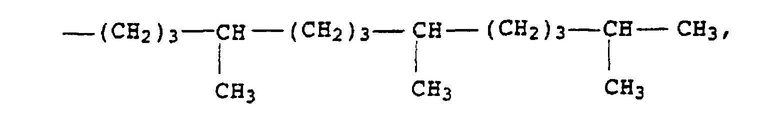 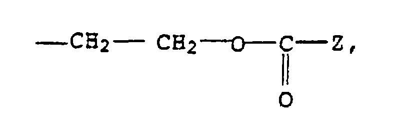 in which Z is C₇- to C₃₀-alkyl,
-CH₂-CH₂-S-(C₁- to C₃₀-alkyl) or 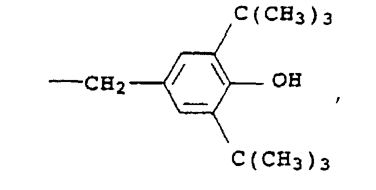
(b) an inert organic solvent for the chroman derivatives I from the group consisting of aliphatic, cycloaliphatic and aromatic hydrocarbons or halogenated hydrocarbons, silicone oils and vegetable and animal fatty oils,
(c) one or more organic phosphites of the general formula II 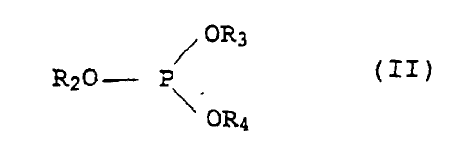 in which the radicals R² to R⁴ are each C₂- to C₁₂-alkyl or C₆- to C₁₈-aryl, which may be substituted by C₁- to C₁₈-alkyl groups,
or an organic phosphonite of the formula III 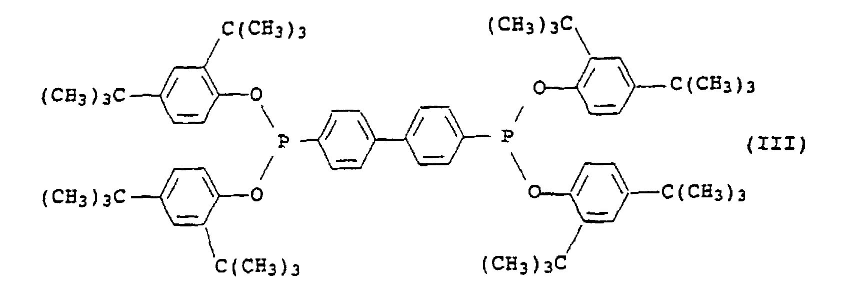 or mixtures of the phosphites II and the phosphonite III, and/or
(d) one or more amines of the general formula IV 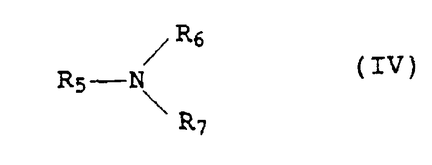 in which the radicals R⁵ to R⁷ are each hydrogen, C₁- to C₁₈-alkyl, which may be interrupted by up to 5 non-adjacent oxygen atoms or groups of the formula -NR⁸- and may be substituted by up to 3 hydroxyl groups,
where R⁸ is hydrogen or C₁- to C₄-alkyl, or are phenyl, which may be substituted by up to 3 C₄- to C₁₈-alkyl groups, with the exception of NH₃ as the amine IV,
where components (a) and (b) are in a weight ratio of from 99:1 to 1:99 and components (b) and (c) are in a weight ratio of from 100:0 to 1:99, and component (d) is present in the stabilizer mixture in an amount of from 0 to 2.0% by weight, based on the amount of (a) + (c) with the proviso that at least one of components (c) or (d) is present.

2. Use of microcapsules as claimed in claim 1 for stabilizing organic material against the action of light, oxygen and heat.

3. Organic material which is stabilized against the action of light, oxygen and heat, comprising from 0.005 to 10.0% by weight, based on the amount of the organic material, of microcapsules as claimed in claim 1.

## Revendications

1. Microcapsules contenant un mélange stabilisateur contenant
(a) un ou plusieurs dérivés de chromane de formule générale I 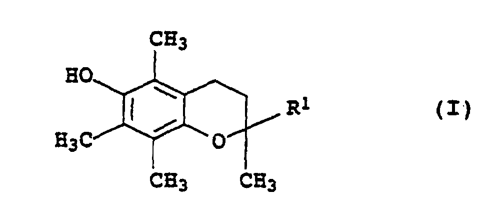 dans laquelle R¹ représente un groupe de formules 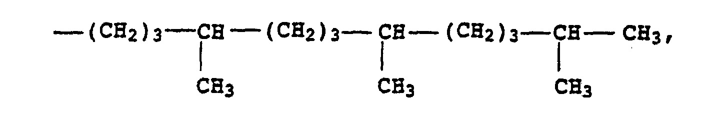 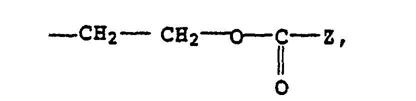 dans laquelle Z représente un groupe alkyle en C₇ à C₃₀, -CH₂CH₂-S-(alkyle en C₁ à C₃₀) ou 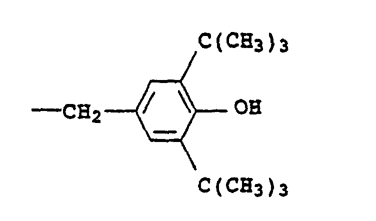
(b) un solvant organique inerte pour les dérivés de chromane I du groupe formé par les hydrocarbures aliphatiques, cycloaliphatiques ou aromatiques ou pour les hydrocarbures halogénés, les huiles de silicones et les huiles grasses végétales et animales,
(c) un ou plusieurs phosphites organiques de formule générale II 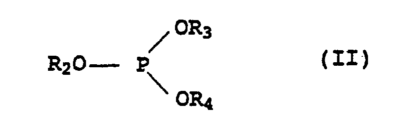 dans laquelle les restes R² à R⁴ représentent chacun un groupe alkyle en C₂ à C₁₂ ou aryle en C₆ à C₁₈ qui est éventuellement substitué par un groupe alkyle en C₁ à C₁₈,
ou un phosphonite organique de formule III 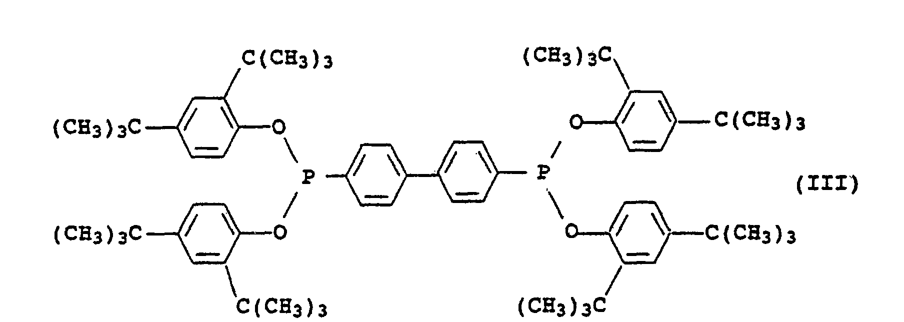 ou un mélange constitué des phosphites II et du phosphonite III et/ou
(d) une ou plusieurs amines de formule générale IV 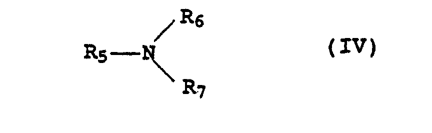
dans laquelle les restes R⁵ à R⁷ représentent chacun un atome d'hydrogène, un reste alkyle en C₁ à C₁₈ qui est éventuellement interrompu par jusqu'à 5 atomes d'oxygène ou groupes de formule -NR⁸- non vicinaux et substitué par jusqu'à 3 groupes hydroxyle, où R⁸ représente un atome d'hydrogène ou un groupe alkyle en C₁ à C₄, ou bien un groupe phényle qui est éventuellement substitué par jusqu'à 3 groupes alkyles en C₄ à C₁₈, à l'exception de NH₃ en tant qu'amine IV,
où le rapport pondéral des composants (a) et (b) est compris entre 99:1 et 1:99, et le rapport pondéral des composants (b) et (c) est compris entre 100:0 et 1:99, et le composant (d) est présent dans le mélange stabilisateur à raison de 0 à 2,0% en poids par rapport à la quantité de (a)+(c), à condition qu'au moins un des composants (c) ou (d) soit présent.

2. Mise en oeuvre des microcapsules selon la revendication 1 pour la stabilisation de matière organique contre l'effet de la lumière, de l'oxygène et de la chaleur.

3. Matière organique stabilisée contre l'effet de la lumière, de l'oxygène et de la chaleur, contenant les microcapsules selon la revendication 1 à raison de 0,005% à 10,0% en poids par rapport à la quantité de matière organique.
